Europäisches Patentamt

European Patent Office

Office européen des brevets

(19)

(11) Publication number: **0 099 209**

A1

(12) **EUROPEAN PATENT APPLICATION**

(21) Application number: 83303799.7

(22) Date of filing: 30.06.83

(51) Int. Cl.³: **A 01 N 47/44**
A 61 L 15/00, A 61 L 2/22
//(A01N47/44, 33/12, 31/02)

(30) Priority: 01.07.82 GB 8219061

(43) Date of publication of application:
25.01.84 Bulletin 84/4

(84) Designated Contracting States:
CH DE FR GB IT LI SE

(71) Applicant: SURGIKOS INC.
501 George Street
New Brunswick New Jersey 08903(US)

(72) Inventor: Coates, David Anthony
5, Park Avenue
Skipton North Yorkshire BD23 1PN(GB)

(72) Inventor: Hardie, Ian Duncan
37 Plewlands Avenue
Edinburgh Scotland(GB)

(74) Representative: Jones, Alan John et al,
CARPMAELS & RANSFORD 43 Bloomsbury Square
London, WC1A 2RA(GB)

(54) Aqueous disinfectant solutions having residual biocidal activity.

(57) The present invention relates to aqueous disinfectant solutions having residual biocidal activity.

The aqueous solutions comprise from 60 to 80% v/v of a $C_1$ to $C_4$ alkanol and at least two anti-microbial agents, the at least two agents having a combined concentration in the solution of up to 2% w/v.

The first antimicrobial agent is a biguanide compound and the second is a quaternary ammonium compound.

Preferably the alkanol is ethanol or isopropanol.

The composition may be impregnated onto a cloth, such as paper or a fabric to form disinfectant wipes or may be used as aerosol sprays. The composition will be useful in cleaning hard surfaces in hospitals. They show good biocidal activity which remains effective after evaporation of the alcohol. The compositions also facilitate removal of soil from the surfaces.

– 1 –

AQUEOUS DISINFECTANT SOLUTIONS HAVING RESIDUAL BIOCIDAL
ACTIVITY

The present invention relates to aqueous disinfectant
solutions having residual biocidal activity which may
be used, for example, in aerosol sprays or impregnated
onto cloths to form wipes.

Wipes impregnated with alkanols, such as ethanol or
isopropanol, are known.  For instance a product sold
under the name "Azo-Wipe" comprises a non-woven fabric
saturated with isopropyl alcohol.  "Azo-wipe" wipes can
be used to wipe hard surfaces but have the disadvantage
that their biocidal activity only remains until the
alkanol has evaporated.  Moreover, the alkanol can cause
blood to congeal and thereby make soil removal difficult.

An aerosol spray formulation for feminine hygiene is
described in British Patent Specification No.1,026,831
to Mediline A.G.  The formulation contains one or preferably two synthetic bactericides and an emollient in
a propellant liquid.  The formulation may contain up
to 5% of a lower alkanol.  The bactericide may be a
quaternary ammonium compound, a halogenophenyl bactericide
or a guanide-based bactericide.  Preferably a mixture
of the first two types are used.  The aerosol is not
indicated to be of use for disinfecting hard surfaces.

United States Patent Specification No.4,311,479, assigned
to Exterma-Germ Products Ltd., describes a cloth impregnated with an antimicrobial composition.  The cloth
is activated for use by contact with water.  Two compositions described comprise mixtures of a quaternary
ammonium compound and a polymeric biguanide.  These
are impregnated onto the cloth as aqueous solutions.

Canadian Patent Specification No. 33,513 granted to Dustlkin Products relates to germicidal paper and describes methods of preparing dry papers having germicidal activity. In use the paper may be used as a dust cloth or saturated with water to wipe surfaces. Antibacterial agents used in the dry papers include quaternary ammonium compounds. The patent does not mention the use of lower alkanols or guanide compounds as anti-bacterial agents, nor the use of liquid compositions as biocidal agents.

An aerosol spray for disinfecting hard surfaces, containing 0.02% 1,1' hexamethylenebis [5-(4-chlorophenyl) biguanide] digluconate (usually known as chlorhexidine gluconate) in 70% industrial methylated spirit (IMS), is presently on the market. The spray has short-term biocidal activity due to its alcohol content, but the chlorhexidine gluconate has only limited activity against Gram negative organisms, especially Pseudomonas species. It is also inactivated in the presence of organic soil.

Other disinfectant compositions are disclosed in British Patent Specifications Nos: 1553 132 (Salkin), 1 499 448 (Arbrook), 1 431 238 (Bayer) 1 268 576 (Goldschmidt), 1 178 757 (Rohner) 1 101 830 (Deutsche Solvay) and 1 046 966 (Armour). However, there is no indication in these specifications that the compositions disclosed therein have significant residual activity. Many of the disclosed compositions rely for their activity on the presence of special components not used in the compositions of the present invention.

According to the present invention, an aqueous disinfectant solution comprises:

a)    60 to 80% v/v of a $C_1$ to $C_4$ alkanol;

b)    a bisguanide antimicrobial agent; and

c)    a quaternary ammonium antimicrobial agent, wherein the combined concentration of the antimicrobial agents in the solution is up to 2% w/v.

Preferably, the combined concentration of the antimicrobial agents is up to 1% w/v.

The disinfectant composition of the present invention may be used to disinfect and remove soil contaminants from hard surfaces and confer a residual biocidal activity thereon.  For example, the compositions in the form of sprays or impregnated wipes may be used for disinfecting or removing soil from hard surfaces, such as operating tables, instruments and machinery in hospitals or surgeries.

The $C_1$ to $C_4$ alkanol may be methanol, ethanol, one of the propanols or a mixture of such alkanols, for instance IMS.  Preferably, the alkanol is isopropanol or ethanol.  Conveniently, the ethanol is denatured.

The alkanol preferably comprises about 70% v/v, of the composition.  The alkanol is itself a biocidal agent.

The biguanide antimicrobial agent is preferably 1,1' hexamethylenebis [5-(4-chlorophenyl) biguanide] digluconate (chlorhexidine gluconate), but may also be 1,1' -hexamethylene bis [5-(4-chlorophenyl)biguanide]diacetate or 1,1'-hexamethylenebis [5-(4-chlorophenyl) biguanide] dihydrochloride.  Alternatively, the biguanide antimicrobial agent may be a soluble salt of 1,6-di-(4-chlorophenyl -biguanide) hexane or a polymeric biguanide sold as Vantocil 1B (Registered Trade Mark).  (Vantocil 1B is manufactured by Imperial Chemical Industries Ltd.).

- 4 -

In a preferred embodiment of the invention, the quaternary ammonium antimicrobial agent comprises:

an alkyldimethylbenzyl -, alkyltrimethyl-, or dialkyldimethyl - ammonium halide or a mixture thereof.

Preferably, the alkyl groups in the quaternary ammonium compounds are straight chain and contain from 12 to 18 carbon atoms. The halide is preferably chloride.

Suitable quaternary ammonium compounds include octyl dimethyl benzyl ammonium chloride, octyl decyl dimethyl ammonium chloride, dioctyl dimethyl ammonium chloride, didecyl dimethyl ammonium chloride and dimethyl ethyl benzyl ammonium chloride.

A particularly suitable quaternary ammonium compound is Bardac 205M, manufactured by Lonza of Basle, Switzerland. It is a mixture of alkyl dimethyl benzyl ammonium chlorides and dialkyl dimethyl ammonium chlorides.

The composition may also include a chelating agent, such as ethylenediaminetetra-acetic acid (EDTA), which are known to render some micro-organisms more susceptible to antimicrobial agents.

The composition may also include a surfactant, preferably a non-ionic surfactant, to facilitate the cleaning of soiled surfaces. A preferred surfactant is a non-ionic surfactant supplied under the trade name Pluronic F88 (supplied by B.A.S.F. Chemicals of Wyandotte, U.S.A.). Alternatively, the surfactant may be an amphoteric surfactant supplied under the trade name Lexaine C (manufactured by Index Chemicals of Philadelphia, U.S.A). The surfactant should be compatible with all the other components of the mixutre. (Lexaine C is not compatible with chlorhexidine gluconate and should therefore not be used in combination therewith).

The compositions of the present invention may also include up to about 0.2% of a fragrance to give the composition a pleasant odour. Suitable fragrances are those supplied by International Flavours and Fragrances Ltd. under the designations EAG 5109, EAG 5110, EAG 5198 and EAG 5199.

Preferably the composition is impregnated onto a cloth such as paper or a woven or non-woven fabric to provide a disinfectant wipe. Advantageously the wipe is saturated with the composition.

Particularly suitable cloths for use in providing a wipe are those free from binders, being bonded either by thermal bonding techniques such as calendering or by processes of the type used in providing spun bonded fabrics.

Alternatively, the composition is mixed with a propellant to provide a formulation suitable for use as an aerosol spray. Conventional propellants and additives, such as mist activators, may be used to provide a sprayable composition. Preferably, the spray can containing the composition is provided with a 360° valve to facilitate cleaning of difficultly-accessible surfaces.

Application of the composition of the present invention to a suitable substrate has an immediate biocidal action on the substrate. The composition has mycobactericidal, fungicidal, virucidal and antibacterial activity. It is effective against both Gram positive and Gram negative bacteria, including Pseudomonas species. Moreover its activity is retained for a considerable time after evaporation of the alkanol. This residual activity is not exhibited by other commercially available compositions.

- 6 -

It is envisaged that the compositions of the present
invention, particularly in the form of disinfectant
wipes or sprays, will be of use in hospitals and surgeries
to clean hard surfaces, such as operating tables and
instrument trays, surgical instruments and machinery.
The effectiveness of the compositions of the present
invention is thought to be due to the use of three
types of biocidal agent contained therein which provide
a broad spectrum of immediate and residual biocidal
activity.

Some preferred embodiments in accordance with the
present invention will now be described by way of
Example.

## EXAMPLE 1

The following ingredients were mixed together

| | |
|---|---|
| Denatured ethanol | 700 ml |
| Bardac 205 M | 2.0g |
| Chlorhexidine gluconate | 2.5g |

The mixture was made up to 1 litre with water.

Alternatively, 12.5ml of chlorhexidine gluconate BP
(a 20% aqueous solution of chlorhexidine gluconate)
can be used in place of the solid chlorhexidine gluconate.

Bardac 205 M has the following composition:

| | |
|---|---|
| Alkyl ($C_{14}$, 50% $C_{12}$, 40%; $C_{16}$, 10%) dimethyl benzyl ammonium chloride | 20% |
| Octyl decyl dimethyl ammonium chloride | 15% |
| Dioctyl dimethyl ammonium chloride | 7.5% |

|                                            |       |
|--------------------------------------------|-------|
| Didecyl dimethyl ammonium chloride         | 7.5%  |
| Inerts                                     | 50%   |

The final composition therefore contained 70% v/v denatured ethanol, 0.25% w/v biguanide antimicrobial agent and 0.1% of quaternary ammonium compounds. If desired, up to 0.2% w/v of a fragrance may be added to the composition.

### EXAMPLE 2

The following ingredients were mixed together:

| | |
|---|---|
| Isopropyl alcohol | 700 ml |
| Bardac 205 M | 0.4g |
| Vantocil 1B | 25 ml |

The mixture was made up to a volume of 1 litre with water.

Vantocil 1B is an aqueous, slightly opalescent, pale yellow liquid containing 20% of a polymeric biguanide hydrochloride. It has a viscosity of 3-5 cP (25°C) and a specific gravity of 1.035 (25°C).

The final composition therefore contained 70% isopropyl alcohol, 0.5% w/v active biguanide compound and 0.02% w/v active quaternary ammonium compounds. If desired, up to 0.2% w/v of a fragrance may be added to the composition.

### EXAMPLE 3

Disinfectant wipes were prepared by dipping a roll of non-woven fabric into the composition of Example 1 or Example 2. The fabric was either a binder free non-woven fabric, or a modified entangled rayon fibre fabric bonded with 5% of Primal HA8 binder supplied by Chicopee Ltd. of New Brunswick U.S.A. The impregnated roll was placed in a hermetically-sealed evaporation-resistant container prior to use.

### EXAMPLE 4

The composition of Example 1 or Example 2 was also mixed with Arcton 12, a fluorocarbon propellant, in a weight ratio of 70:30. and placed under pressure in an aerosol spray can be arranged to produce a fine mist. The aerosol mixture was prepared and placed in a pressurised container using conventional techniques and additives. The container was provided with a 360° valve.

### EXPERIMENTAL RESULTS

A hard surface was simulated by use of a 15 cm x 15 cm white ceramic tile. The surface was divided into two equal portions by a line drawn thereon, and both portions were contaminated with bacteria by spraying, using a hand atomiser. The spray contained a test organism at a concentration of between $10^7$ and $10^8$ organisms per ml. Tests were carried out using <u>Pseudomonas aeruginosa, Staph. aureus, E coli</u> and <u>Salmonella cholera-eusis.</u>

The tile was allowed to dry in air for about 10 minutes. It was shown that the dried tiles had approximately $8 \times 10^5$ organisms per tile ($3.5 \times 10^3$ organisms per $cm^2$).

One portion of the tile was treated by either wiping or spraying with a disinfectant composition. In the case of spraying, care was taken to ensure that the untreated portion of the tile was fully covered. The tile was again allowed to dry in air for up to 10 minutes. Samples were taken from the treated and untreated poritons of the tile by taking RODAC plate impressions. The RODAC plates contained 0.75% TWEEN 80 (Registered Trade Mark) and 0.75% lecithin. The TWEEN 80 and lecithin act to neutralise the activity of any biocidal compounds in the sample and are used following the procedure of Babb et al (J. of Hosp. Inf., 2, 267-272).

The tile was then recontaminated over the whole area by respraying from the hand atomiser. The tile was allowed to dry in air for approximately 10 minutes. Samples were again taken from the treated and untreated portions and plated using RODAC plates. All the plates were incubated at 32°C. for 48 hours.

The plates covered with samples from the untreated portions are called Control 1 and Control 2 and the plates covered with samples from the treated portions are called Treatment and Residual in the following Table 1.

The results given in Table 1 are for use of Pseudomonas aeruginosa, but similar results have been obtained using other organisms.

In Table 1, + indicates that confluent growth of the organism was observed, - indicates no growth or a few isolated colonies of growth was observed, Test 1 refers to wiping with an Azo-Wipe wipe (a wipe saturated in 70% isopropyl alcohol), Test 2 to spraying with DISPRAY 2, (a commercially-available spray containing 0.02% chlorhexidine gluconate in 70% IMS), Test 3 to wiping with a wipe including a composition according to the present invention and Test 4 to spray with a composition according to the invention. In Tests 3a and 4a, a composition according to Example 1 was used and in Tests 3b and 4b, a composition according to Example 2 was used.

In tests 3a or b -I, the binder free fabric was used and in Tests 3a or b - II, the bound fabric was used.

TABLE 1

| Test | Control 1 | Control 2 | Treatment | Residual |
|------|-----------|-----------|-----------|----------|
| 1 | + | + | − | + |
| 2 | + | + | − | + |
| 3a-I | + | + | − | − |
| 3a-II | + | + | − | − |
| 3b-I | + | + | − | − |
| 3b-II | + | + | − | − |
| 4a | + | + | − | − |
| 4b | + | + | − | − |

It can be seen from the Table that presently-available wipes or sprays are able to confer immediate protection on a hard surface, but this protection is short-lived, whereas wipes or sprays including compositions according to the present invention confer on the surface a residual biocidal activity. This residual activity can last for up to 24 hours.

It is possible that, in the above test procedure, the inactivation effect of the TWEEN 80 and lecithin may not be immediate due to the time needed to complete inactivation. Therefore, to confirm the residual activity of the compositions of the present invention the following test was employed. It is derived from Lowbury and Lilly's technique for determining the number of viable bacteria removed from hands when washing. (B.M.J., May 14, 1960, 1448-1450).

A ceramic white tile (15 cm x 15 cm) was treated with a biocide formulation, either by wiping or spraying, and allowed to dry and stand for a certain time. Thereafter a carefully controlled number of organisms was sprayed onto the tile. The tile was placed in a sterile polyethylene bag containing 100 ml of a neutraliser solution (1% TWEEN 80, 1% lecithin). After 1 minute of uniform gentle agitation, 1 ml aliquots were removed, diluted in phosphate buffer and plated out with molten tryptone soya agar. The viable number of surviving organisms can be calculated after incubation at 32°C for 48 hours.

The numbers of viable organisms recovered from the tile treated with a wipe composition according to the present invention can be calculated as % survivors against those found with the commercially available Azo-Wipe or Dispray 2, i.e. % survivors is equal to

$$\frac{\text{number viable using a composition according to the present invention}}{\text{number viable using either Azo-Wipe or Dispray 2}} \times 100$$

Table 2 shows the results obtained using a wipe of the present invention (incorporating a composition according to Example 2) against commercially available products, with various reinfecting organisms.

The composition according to Example 1 has very similar activity to that of Example 2 and therefore a test using a wipe incorporating a composition according to Example 1 against the organisms given in Table 2 would yield results closely similar to those given in Table 2.

TABLE 2

NUMBER (ORGS/ML.) AND % SURVIVORS

| ORGANISM | ASOWIPE | V | WIPE* | DISPRAY 2 | V | WIPE |
|---|---|---|---|---|---|---|
| Staph. aureus | $3.5 \times 10^5$ 100% | v | $2.9 \times 10^4$ 8.2% | $2.1 \times 10^6$ 100% | v | $6.6 \times 10^3$ 0.3% |
| Salm. cholera | $2.1 \times 10^5$ 100% | v | $2.3 \times 10^3$ 1% | $1.1 \times 10^5$ 100% | v | $8.8 \times 10^1$ 0.1% |
| Ps. aeruginosa | $4.5 \times 10^5$ 100% | v | $9.2 \times 10^3$ 6.1% | $6.9 \times 10^5$ 100% | v | $2.2 \times 10^3$ 0.4% |

* = An impregnated wipe according to the present invention.

This confirms the previous results, and shows that the composition according to the present invention confers a residual biocidal activity relative to the commercially available products.

If a surfactant, such as Pluronic F 88 or Lexaine C, is included in the composition, cleaning of soiled surfaces is facilitated.

It was shown that the composition of the present invention is active against a wide range of bacteria, including Pseudomonas varieties, by standard tests.

The active ingredients in the composition according to the invention have proved histories of low toxicity. Therefore its use should present no problems from this point of view. Moreover, the composition may be used, without adverse effects, on a wide variety of substrates, such as most metals, adequately-compounded rubbers and plastics. However, biguanide compounds may tarnish copper and it may be advisable not to use the present composition on copper utensils or surfaces.

Thus the present invention provides a composition which can be used to confer residual biocidal activity on a disinfected surface.

CLAIMS

1. An aqueous disinfectant solution comprising:

a) 60 to 80% v/v of a $C_1$ to $C_4$ alkanol;

b) a biguanide antimicrobial agent; and

c) a quaternary ammonium antimicrobia agent, wherein the combined concentration of the antimicrobial agents in the solution is up to 2% w/v.

2. An aqueous disinfectant solution according to claim 1, wherein the combined concentration is up to 1% w/v.

3. An aqueous disinfectant solution according to claim 1 or claim 2, wherein the alkanol is ethanol or isopropanol.

4. An aqueous disinfectant solution according to claim 3, wherein the alkanol is denatured ethanol.

5. An aqueous disinfectant solution according to any one of claims 1 to 4, comprising about 70% v/v of the alkanol.

6. An aqueous disinfectant solution according to any one of claims 1 to 5 wherein the biguanide antimicrobial agent is 1,1' hexamethylenebis [5-(4-chlorophenyl) biguanide] digluconate.

7. An aqueous disinfectant solution according to any one of claims 1 to 5, wherein the biguanide antimicrobial agent is a polymeric biguanide.

8. An aqueous disinfectant solution according to any one of claims 1 to 7, wherein the quaternary ammonium antimicrobial agent comprises an alkyldimethylbenzyl-, alkyltrimethyl-, or dialkyldimethyl- ammonium halide or a mixture thereof.

9. An aqueous disinfectant solution according to claim 8, wherein the alkyl groups in the quaternary ammonium compounds are straight chain and have from 12 to 18 carbon atoms.

10. An aqueous disinfectant solution according to claim 8 or claim 9, wherein the halide is chloride.

11. An aqueous disinfectant solution according to any one of claims 1 to 10, and including a chelating agent.

12. An aqueous disinfectant solution according to any one of claims 1 to 11, and including a surfactant.

13. An aqueous disinfectant solution according to any one of claims 1 to 12, and including up to 0.2% w/v of a fragrance.

14. A disinfectant wipe comprising a cloth impregnated with a solution according to any one of claims 1 to 13.

15. A disinfectant wipe according to claim 14, wherein the cloth is saturated with the solution.

16. A disinfectant wipe according to claim 14 or claim 15, wherein the cloth is a non-woven fabric free of binders.

17. A disinfectant formulation for use in an aerosol spray, comprising a solution according to any one of claims 1 to 13 in admixture with a propellant.

18. A disinfectant aerosol spray comprising a formulation according to claim 17 contained under pressure in an aerosol can.

19.  A method of disinfecting a substrate comprising applying thereto a solution according to any one of claims 1 to 12.

20.  A method according to claim 19, wherein the solution is applied to the substrate using a wipe according to any one of claims 14 to 16.

21.  A method according to claim 19, wherein the solution is applied to the substrate using an aerosol spray according to claim 18.

0099209

European Patent
Office

**EUROPEAN SEARCH REPORT**

Application number

EP 83 30 3799

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,A | US-A-4 311 479  (D.J. FENN et al.)<br><br>* Whole document *<br><br>--- | 1-3,6-10,12 14-16 19,20 | A 01 N   47/44<br>A 61 L   15/00<br>A 61 L    2/22  //<br>(A 01 N   47/44<br>A 01 N   33/12<br>A 01 N   31/02  ) |
| D,A | GB-A-1 553 132  (N. SALKIN)<br><br>*  Page 1, line 90 - page 2, line 63;  page 3, lines 65-80; example 3 *<br><br>--- | 1-6,12 ,13 | |
| A | GB-A-  821 113  (I.C.I.)<br><br><br>*  Page 2, line 77 - page 3, line 65; examples 3,5,8; claims *<br><br>--- | 1-6,8 9,14, 15,19 20 | |
| A | FR-A-2 483 177  (N. SALKIN)<br><br>*  Page 2, line 25 - page 3, line 12; examples *<br><br>----- | 1-5,7-10 | **TECHNICAL FIELDS SEARCHED (Int. Cl. 3)**<br><br>A 01 N<br>A 61 L |

The present search report has been drawn up for all claims

| Place of search<br>THE HAGUE | Date of completion of the search<br>14-10-1983 | Examiner<br>FLETCHER A.S. |
|---|---|---|

EPO Form 1503 03 82

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document